# EUROPÄISCHE PATENTSCHRIFT

(11) **EP 3 085 398 B1**
(45) Veröffentlichungstag und Bekanntmachung des Hinweises auf die Patenterteilung: **07.08.2019**
(21) Anmeldenummer: 15164983.7
(22) Anmeldetag: 24.04.2015
(51) Int. Cl.: A61M 1/16

(54) **VERFAHREN ZUM DESINFIZIEREN EINER DIALYSATSEITE EINES HÄMODIALYSEGERÄTES**
METHOD FOR DISINFECTING A DIALYSATE SIDE OF A HAEMODIALYSIS UNIT
PROCÉDÉ DE DÉSINFECTION D'UN DIALYSAT D'UN APPAREIL D'HÉMODIALYSE

(43) Veröffentlichungstag der Anmeldung: 26.10.2016
(73) Patentinhaber: D.Med Consulting GmbH, 20359 Hamburg (DE)
(72) Erfinder: Breuch, Gerd, 53844 Troisdorf (DE); Biermann, Frank, 22455 Hamburg (DE); Breuch, Julius, 20457 Hamburg (DE); Holzschuh, Robert, 22301 Hamburg (DE)
(74) Vertreter: Patentanwälte ter Smitten Eberlein-Van Hoof Rütten Partnerschaftsgesellschaft mbB

(56) Entgegenhaltungen:
- DE-A1- 4 138 140
- US-A- 5 895 578
- US-A1- 2009 012 450
- US-A1- 2014 248 600

## Beschreibung

Die Erfindung bezieht sich auf ein Verfahren zum Desinfizieren einer Dialysatseite eines Hämodialysegerätes.

Nach jeder Dialysebehandlung muss die geräteseitige Dialysatseite des Hämodialysegeräts gereinigt und desinfiziert werden, um für die folgende Hämodialysebehandlung wieder in einen keimfreien Zustand versetzt zu werden. Unter der Dialysatseite sind alle geräteseitigen Flüssigkeitsleitungen zu verstehen, die nach einer Hämodialysebehandlung nicht ausgetauscht werden, sondern fest und dauerhaft in bzw. an dem Hämodialysegerät verbaut sind, und daher zum Schutz des jeweils folgenden Hämodialyse-Patienten praktisch keimfrei gemacht werden müssen, um eine Infektion der folgenden Patienten auf diesem Wege zu vermeiden. Die Dialysatseite eines Hämodialysegerätes weist unter anderem eine Dialysator-Zulaufleitung, die im Dialysebetrieb eine Dialysatquelle mit einem Zulaufkonnektor fluidisch verbindet, und eine Dialysator-Ablaufleitung auf, die im Dialysebetrieb einen Ablaufkonnektor mit einem Abfallpfad fluidisch verbindet, in den das verbrauchte Dialysat während der Dialysebehandlung abgeführt wird. An den Zulaufkonnektor und den Ablaufkonnektor ist während einer Dialysebehandlung ein Dialysator mit entsprechenden dialysatorseitigen Konnektoren fluidisch angeschlossen, in dem die harnpflichtigen Substanzen aus dem in einem extrakorporalen Blutkreislauf fließenden Patientenblut in die den Dialysator durchströmende Dialyseflüssigkeit gelangen.

Für die Desinfektion nach einer Dialysebehandlung kommt die physikalische Desinfektion, insbesondere durch Erhitzung, und die chemische Desinfektion in Frage. Bei der chemischen Desinfektion wird zunächst ein geschlossener Fluidkreis dadurch hergestellt, dass der Dialysator durch Trennen der beiden Konnektor-Verbindungen entfernt wird, und die beiden geräteseitigen Konnektoren durch eine temporäre Rückschlussleitung miteinander verbunden werden. Die temporäre Rückschlussleitung kann ein Wegwerfartikel sein, kann jedoch auch ein mehrfach benutzbarer Artikel sein. Die temporäre Rückschlussleitung kann auch fest in das Hämodialysegerät eingebaut sein. Mit 'temporär' ist vorliegend in erster Linie die temporäre Nutzung gemeint, jedoch nicht notwendigerweise die nur temporäre Anwesenheit in bzw. an dem Hämodialysegerät.

Zum Schließen des Fluidkreises ist ferner eine Rezirkulations-Leitung vorgesehen, die die Dialysator-Zulaufleitung stromaufwärts des Zulaufkonnektors und die Dialysator-Ablaufleitung stromabwärts des Ablaufkonnektors fluidisch miteinander verbindet. In der Rezirkulations-Leitung kann ein schaltbares Rezirkulations-Ventil angeordnet sein, das während eines Desinfektionszyklus geöffnet ist.

Zur Desinfektion wird eine Desinfektionsflüssigkeit in den geschlossenen Fluidkreis eingeleitet und für eine festgelegte Desinfektionszeit in dem Fluidkreis im Kreis gepumpt. Typische Desinfektionsflüssigkeiten sind beispielsweise Peressigsäure, Chlor, Zitronensäure et cetera. Nach Ablauf der festgelegten Desinfektionszeit, die beispielsweise 20 Minuten beträgt, wird die Desinfektionsflüssigkeit in den Abfallpfad abgepumpt. Anschließend wird der Fluidkreis mit Leitungswasser oder Dialysewasser gefüllt, wo es bis zur folgenden Hämodialysebehandlung in dem Fluidkreis verbleibt.

Die Keimbelastung von Wasser verdoppelt sich bei Raumtemperatur nach jeweils ca. 20 min, was insbesondere problematisch ist, wenn die folgende Hämodialysebehandlung erst nach mehreren Tagen erfolgt, wie dies in der Praxis über ein Wochenende auftreten kann. Nach spätestens 72 Stunden ohne Desinfektion muss vor einer Hämodialysebehandlung daher eine erneute Desinfektion der geräteseitigen Leitungen erfolgen. Dies ist kostspielig, zeitraubend und umständlich.

Aus DE 41 38 140 C2 ist ein Desinfektions-Verfahren bekannt, bei dem nach der eigentlichen Desinfektion lediglich die Zirkulation der Desinfektionsflüssigkeit in dem Fluidkreis gestoppt wird, so dass die Desinfektionsflüssigkeit bis zur folgenden Hämodialysebehandlung in dem Fluidkreis stehen bleibt. Vor der folgenden Hämodialysebehandlung wird der Fluidkreis mit Wasser freigespült. Zwar wird durch dieses Verfahren auch nach einer langen Stillstandszeit sichergestellt, dass die betreffenden Fluldleitungen nach dem Freispülen keimfrei sind. Nachteilig ist jedoch, dass durch die aggressive Desinfektionsflüssigkeit Korrosion in dem Fluidkreis befördert wird und dass zur sicheren Vermeidung von kleinen Residualvolumina der gegebenenfalls gesundheitsschädlichen Desinfektionsflüssigkeit das Freispülen vor einer folgenden Hämodialysebehandlung relativ langwierig ist.

Aus US 5,895,578 A ist ein Desinfektionsverfahren bekannt, bei dem nach der Desinfektion die Desinfektionsflüssigkeit abgelassen wird, und durch eine Konservierungsflüssigkeit ersetzt wird.

Aufgabe der Erfindung vor diesem Hintergrund ist es, ein effizienteres Verfahren zum Desinfizieren der Dialysatseite eines Hämodialysegeräts zu schaffen.

Diese Aufgabe wird erfindungsgemäß mit einem Verfahren zum Desinfizieren der Dialysatseite eines Hämodialysegeräts mit den Merkmalen des Anspruchs 1 gelöst.

Das Hämodialysegerät weist eine Dialysator-Zulaufleitung auf, die im Dialysebetrieb eine Dialysatflüssigkeits-Quelle mit einem Zulaufkonnektor fluidisch verbindet, und weist eine Dialysator-Ablaufleitung auf, die fluidisch einen Ablaufkonnektor mit einem Abfallpfad verbindet, der beispielsweise in einen Abfallbehälter mündet, in dem die Abfallflüssigkeit gesammelt wird. An den Zulaufkonnektor und an den Ablaufkonnektor sind während des Dialysebetriebs entsprechende Konnektoren des Dialysators angeschlossen, die fluidisch mit der Dialysatseite des Dialysators verbunden sind.

Für das erfindungsgemäße Desinfektionsverfahren ist an die Konnektoren eine temporäre Rückschlussleitung angeschlossen, die die beiden geräteseitigen Konnektoren fluidisch miteinander verbindet. Die temporäre Rückschlussleitung kann geräteseitig auch fest installiert und während des erfindungsgemäßen Desinfektionsverfahrens nur vorübergehend durchlässig sein.

Ferner ist zwischen der Dialysator-Zulaufleitung und der Dialysator-Ablaufleitung eine separate Rezirkulations-Leitung vorgesehen, die das stromaufwärts-seitige Ende der Zulaufleitung und das stromabwärtsseitige Ende der Ablaufleitung fluidisch miteinander verbindet. Im Verlauf der Rezirkulationsleitung ist ein schaltbares Rezirkulations-Ventil angeordnet, durch das die Rezirkulationsleitung geöffnet oder geschlossen werden kann. Die Rezirkulationsleitung ist bevorzugt geräteseitig fest installiert, also nicht als Wegwerfartikel ausgelegt.

Im Verlauf der Dialysator-Zulaufleitung und/oder im Verlauf der Dialysator-Ablaufleitung und/oder im Verlauf einer der beiden übrigen Leitungen ist eine Flüssigkeitspumpe angeordnet, die besonders bevorzugt als Dialysatpumpe im Verlauf der Dialysator-Zulaufleitung oder als Abfallpumpe im Verlauf der Dialysator-Ablaufleitung ausgebildet sein kann.

Zunächst wird die Desinfektionsflüssigkeit in eine der vorgenannten Leitungen eingeleitet, bevorzugt über ein hierzu geöffnetes Desinfektionsventil. Die Desinfektionsflüssigkeit kommt von einem Desinfektionsflüssigkeit-Behälter, der beispielsweise ein Beutel sein kann. Die Desinfektionsflüssigkeit kann auch aus einem Pulver, das vor Ort in Dialysewasser gelöst wird, generiert werden.

Gemäß den Verfahrensschritten nach Anspruch 1 wird das Rezirkulationsventil geöffnet und wird die Flüssigkeitspumpe aktiviert, so dass die Desinfektionsflüssigkeit in die zu einem Fluidkreis zusammengebundenen Leitungen eingeleitet wird. Zum Einleiten der Desinfektionsflüssigkeit kann ferner, soweit vorhanden, ein Desinfektionsventil geöffnet werden. Sobald der gesamte von den vorgenannten Leitungen gebildete Fluidkreis vollständig mit der Desinfektionsflüssigkeit gefüllt ist, kann das Desinfektionsventil wieder geschlossen werden, so dass die Desinfektionsflüssigkeit in dem Fluidkreis für die Dauer der eingestellten Desinfektionszeit von beispielsweise 20 Minuten kontinuierlich zirkuliert. Nach Ablauf der Desinfektionszeit kann davon ausgegangen werden, dass der Fluidkreis, also insbesondere die Dialysator-Zulaufleitung und die Dialysator-Ablaufleitung, vollständig desinfiziert ist.

Nach Ablauf der eingestellten Desinfektionszeit wird das Konservierungsventil geöffnet, wodurch die Konservierungsflüssigkeit in alle Leitungen des Fluidkreises eingeleitet wird. Gleichzeitig wird der Abfallpfad fluidisch stromabwärts der Dialysator-Ablaufleitung geöffnet, so dass die Flüssigkeit aus der Dialysator-Ablaufleitung teilweise in den Abfallpfad und teilweise durch die Rezirkulations-Leitung fließt. Hierdurch wird die Desinfektionsflüssigkeit in dem Fluidkreis kontinuierlich verdünnt, bis die gewünschte Verdünnung der Desinfektionsflüssigkeit, die größer als 1% ist, erreicht ist. Sobald die gewünschte Verdünnung der Desinfektionsflüssigkeit erreicht ist, werden das Konservierungsventil und das gegebenenfalls vorhandene Abfallventil wieder geschlossen, so dass die Desinfektionsflüssigkeit nicht weiter verdünnt wird und keine Flüssigkeit mehr in den Abfallpfad abfließt. Die Flüssigkeit wird vorzugsweise weiter im Kreis gepumpt, bis die Konzentration bzw. Verdünnung homogenisiert ist, so dass sie an jeder Stelle des Kreises gleich ist.

Die Konservierungsflüssigkeit ist ein Verdünnungsmittel, mit dem die Desinfektionsflüssigkeit in den Leitungen des Fluidkreises auf höchstens 50 %, bevorzugt auf höchstens 30 %, und in der Praxis auf ungefähr 20-10 % verdünnt wird. In jedem Fall liegt die Verdünnung über 1%.

Besonders bevorzugt ist die Konservierungsflüssigkeit Dialysewasser das während der Dialysebehandlung der Erzeugung der Dialyseflüssigkeit dient. Die durch das Verdünnungsmittel erheblich verdünnte Desinfektionsflüssigkeit wird auf diese Weise zu einem Konservierungsmittel, das einerseits die Keimbildung erheblich oder nahezu vollständig hemmt und andererseits nur geringe Korrosionskraft hat. Wegen der relativ geringen Konzentration der Desinfektionsflüssigkeit sind Residualvolumina der Konservierungsflüssigkeit bezüglich der Gesundheitsgefährdung nicht mehr so kritisch, wie dies für eine unverdünnte Desinfektionsflüssigkeit der Fall wäre.

Während des Konservierungszyklus' wird die Flüssigkeitspumpe bevorzugt angehalten und werden die Ventile innerhalb des Fluidkreises bevorzugt geschlossen. Besonders bevorzugt werden insbesondere das Desinfektionsventil, das Konservierungsventil und das Rezirkulations-Ventil während der Dauer des Konservierungszyklus' geschlossen.

Der erfindungsgemäße Desinfektions- und Konservierungszyklus kann manuell über eine Konservierungstaste des Hämodialysegeräts ausgelöst werden. Alternativ oder ergänzend ist auch eine intelligente automatische Auslösung des erfindungsgemäßen Desinfektions- und Konservierungszyklus möglich, beispielsweise dann, wenn eine Desinfektion freitags nach 16:00 Uhr gestartet wird.

Nach Ablauf der Konservierungszeit wird die Konservierungsflüssigkeit aus den Leitungen des Fluidkreises in den Abfallpfad bzw. die Abfallleitung gepumpt, wofür die Flüssigkeitspumpe aktiviert und die betreffenden Ventile, insbesondere ein Abfallventil, das die Dialysator-Ablaufleitung mit dem Abfallpfad verbindet, geöffnet werden.

Vorzugsweise werden schon während, jedoch in jedem Fall nach dem Abpumpen der Konservierungsflüssigkeit in die Abfallleitung die Leitungen des Fluidkreises mit einer Spülflüssigkeit gespült. Die Spülflüssigkeit ist bevorzugt Dialysewasser, das auch der Erzeugung einer Dialyseflüssigkeit dient.

Das Hämodialysegerät weist einen Desinfektionsflüssigkeits-Behälter auf, in dem die Desinfektionsflüssigkeit gelagert ist. Der Desinfektionsflüssigkeits- Behälter und das sich fluidisch daran anschließende Desinfektionsventil können der temporären Rückschlussleitung fluidisch zugeordnet sein, so dass die Desinfektionsflüssigkeit bei geöffneten Desinfektionsventil in die temporäre Rückschlussleitung eingeleitet wird.

Alternativ hierzu können der Desinfektionsflüssigkeits-Behälter und das Desinfektionsventil der Dialysator-Ablaufleitung zugeordnet sein. Zu der Dialysator-Ablaufleitung wird vorliegend auch ein Parallelpfad zu der eigentlichen Ablaufleitung gezählt, der als Ultrafiltrations-Leitung mit einer in beiden Flussrichtungen betreibbaren Ultrafiltrationspumpe ausgebildet sein kann.

Gemäß einer bevorzugten Ausführungsform kann ein Konzentrations-Sensor im Verlauf einer der Leitungen angeordnet sein, und ist besonders bevorzugt im Verlauf der Dialysator-Zulaufleitung angeordnet. Der Konzentrations-Sensor dient der Bestimmung der Konzentration der Desinfektionsflüssigkeit in der Konservierungsflüssigkeit. Mit dem Konzentrations-Sensor kann die Einleitung der Konservierungsflüssigkeit bzw. des Verdünnungsmittels so geregelt werden, dass eine Soll-Konzentration der Desinfektionsflüssigkeit in dem Verdünnungsmittel eingestellt werden kann.

Vorzugsweise weist das Hämodialysegerät eine Konservierungstaste auf, durch deren Betätigung das Verfahren zum Desinfizieren und Konservieren manuell ausgelöst wird. Die Konservierungstaste kann als mechanischer Taster, als berührungsempfindlichen Taster oder als eine Tastendarstellung auf einem Touchscreen realisiert sein.

Vorzugsweise weist das Hämodialysegerät im Verlauf der Dialysator-Zulaufleitung eine Flüssigkeitsheizung auf, durch die die vorbeilaufende Flüssigkeit aufgeheizt werden kann. Vorzugsweise ist die Flüssigkeitsheizung während der Desinfektionszeit aktiviert, so dass die in dem Fluidkreis zirkulierende Desinfektionsflüssigkeit auf eine Desinfektionstemperatur aufgewärmt wird, die je nach eingesetztem Desinfektionsmittel beispielsweise 50° bis 80 °C betragen kann. Hierdurch wird die Desinfektion erheblich beschleunigt.

Im Folgenden werden zwei Ausführungsbeispiele der Erfindung anhand der Zeichnungen näher erläutert. Es zeigen:
Figur 1 eine erste Ausführungsform eines Hämodialysegeräts, mit dem das erfindungsgemäße Verfahren zum Desinfizieren und Konservieren der Dialysatseite eines Hämodialysegerätes durchgeführt wird, und
Figur 2 eine zweite Ausführungsform eines Hämodialysegerätes, mit dem das erfindungsgemäße Verfahren zum Desinfizieren und Konservieren der Dialysatseite des Hämodialysegeräts durchgeführt wird.

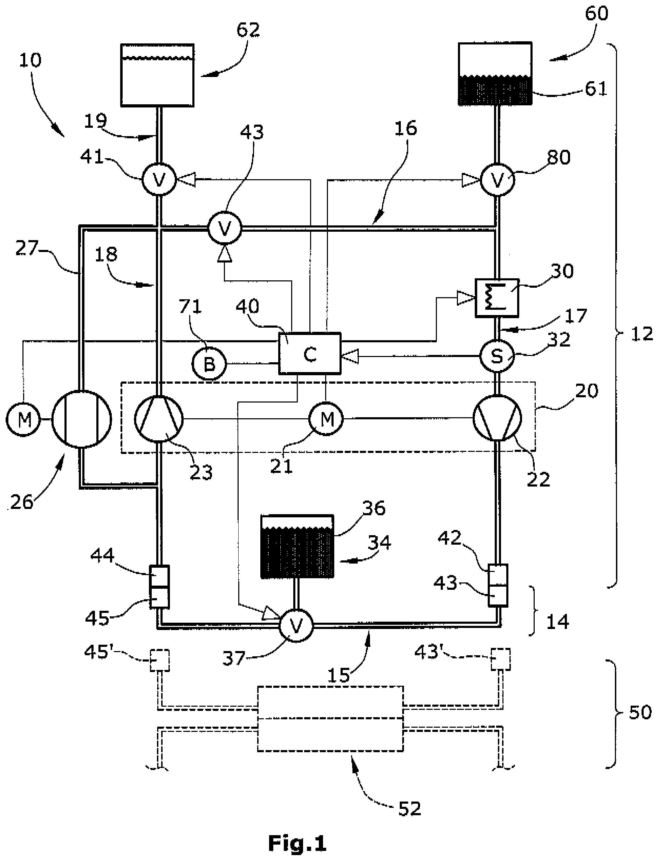

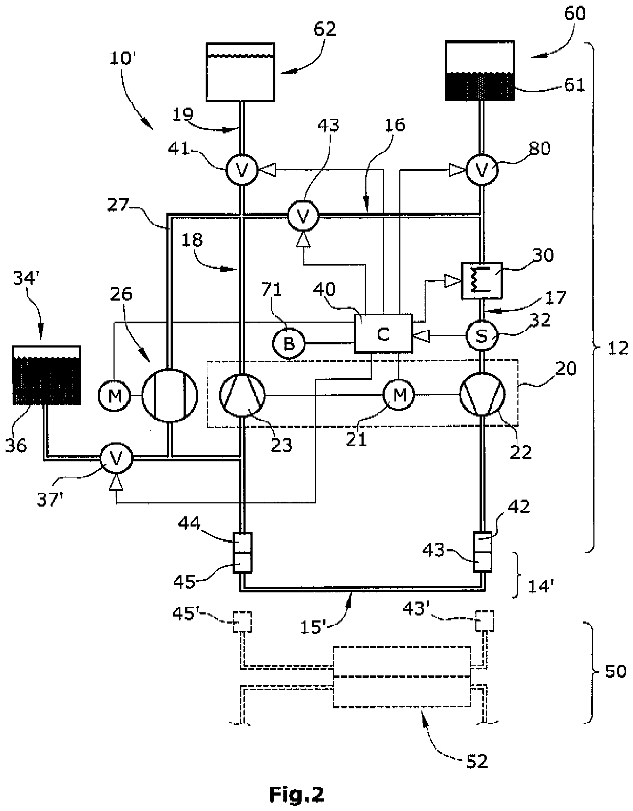

In den Figuren 1 und 2 ist jeweils ein Hämodialysegerät 10;10' dargestellt, das eine geräteseitige Dialysatseite 12 aufweist, die während einer Dialysebehandlung der Versorgung eines Hämodialyse-Dialysators 52 mit einer Dialyseflüssigkeit dient. Die Dialysatseite 12 weist eine Dialysator-Zulaufleitung 17 auf, die sich von einer nicht dargestellten Dialyseflüssigkeits-Quelle bis zu einem Zulaufkonnektor 42 erstreckt. Im Verlauf der Dialysator-Zulaufleitung 17 sind eine Flüssigkeitsheizung 30 zum Erwärmen der vorbeifließenden Flüssigkeit, ein Konzentrations-Sensor 32 zur Bestimmung der Konzentration der Desinfektionsflüssigkeit und eine Flüssigkeitspumpe 22 zum Fördern der Flüssigkeit angeordnet. Die Dialysatseite 12 weist ferner eine Dialysator-Ablaufleitung 18 auf, in deren Verlauf eine Abfallpumpe 23 angeordnet ist. Die Flüssigkeitspumpe 22 und die Abfallpumpe 23 können, müssen jedoch nicht, mechanisch oder elektronisch zu einer Bilanzierpumpe 20 gekoppelt sein. Fluidisch parallel zu der Abfallpumpe 23 ist eine Ultrafiltrationsleitung 27 vorgesehen, in deren Verlauf eine bidirektional betreibbare Ultrafiltrationspumpe 26 angeordnet ist.

Die Dialysator-Zulaufleitung 17 beginnt fluidisch an einem Konservierungsventil 80, an das sich fluidisch stromaufwärts ein Behälter 60 mit einer Konservierungsflüssigkeit 61 anschließt. Der Konservierungsflüssigkeits-Behälter 60 wird nach einer Dialysebehandlung über entsprechende Kupplungen fluidisch mit der Zulaufleitung 17 verbunden, kann jedoch auch bereits während der vorangegangenen Dialysebehandlung angekoppelt gewesen sein und Dialysewasser enthalten. Die Dialysator-Ablaufleitung 18 endet fluidisch an einem Abfallventil 41, an das fluidisch stromabwärts ein Abfallpfad 19 angeschlossen ist, der in einen Abfalltank 62 mündet, in dem der Flüssigabfall gesammelt wird.

Fluidisch zwischen der Zulaufleitung 17 und der Ablaufleitung 18 ist geräteseitig eine Rezirkulationsleitung 16 vorgesehen, in deren Verlauf ein Rezirkulationsventil 43 angeordnet ist, durch das die Rezirkulationsleitung 16 geöffnet oder gesperrt werden kann. Die Rezirkulationsleitung 16 verbindet das stromaufwärts-seitige Ende der Zulaufleitung 17, das an dem Konservierungsventil 80 beginnt, mit dem stromabwärts-seitigen Ende der Ablaufleitung 18, das an dem Abfallventil 41 endet.

Die Zulaufleitung 17, die Ablaufleitung 18, die Ultrafiltrationsleitung 27 und die Rezirkulationsleitung 16 sind geräteseitig fest verbaut, sind also nicht als Wegwerfartikel ausgebildet. Diese Leitungen 16-18,27 müssen daher vor der folgenden Dialysebehandlung desinfiziert werden.

In beiden in den Figuren 1 und 2 dargestellten Ausführungsbeispielen werden während des Desinfizierens und Konservierens der Dialysatseite 12 die beiden geräteseitigen Konnektoren 42,44 durch ein Wegwerf-Rückschlussset 14; 14' fluidisch miteinander verbunden, das jeweils eine temporäre Rückschlussleitung 15; 15' aufweist. An den Enden der Rückschlussleitung 15; 15' ist jeweils ein Konnektor 43,45 angebracht, der mit den korrespondierenden geräteseitigen Konnektoren 42,44 zusammengekoppelt ist.

In dem in der Figur 1 dargestellten ersten Ausführungsbeispiel weist das Rückschlussset 14 ferner ein Desinfektionsventil 37 auf, durch das eine Desinfektionsflüssigkeit 36 aus einem Desinfektionsflüssigkeits-Behälter 34 in die Rückschlussleitung 15 eingeleitet werden kann. Grundsätzlich kann die Einleitung aber auch woanders auf der Dialysatseite erfolgen, z.B. im Bereich der Einleitung der Konservierungsflüssigkeit.

In dem in der Figur 2 dargestellten zweiten Ausführungsbeispiel weist das Rückschlussset 14' keine weiteren Elemente auf, die erfindungswesentlich sind. Die Einleitung der Desinfektionsflüssigkeit 36 erfolgt bei diesem Ausführungsbeispiel in die Dialysator-Ablaufleitung 18 bzw. in die Ultrafiltrationsleitung 27 über ein Desinfektionsventil 37', durch das die Desinfektionsflüssigkeit 36 aus einem Desinfektionsflüssigkeit-Behälter 34' in die Ablaufleitung 18 eingeleitet werden kann.

Als Desinfektionsflüssigkeit 36 dient beispielsweise Peressigsäure, Chlor oder andere geeignete Flüssigkeiten.

Die Konservierungsflüssigkeit 61 ist vorliegend ein Verdünnungsmittel, beispielsweise Dialysewasser, das auch zur Erzeugung der Dialyseflüssigkeit bei der vorangegangenen Dialysebehandlung genutzt wurde. Grundsätzlich ist jedoch auch jede andere Flüssigkeit geeignet, die zur Verdünnung der Desinfektionsflüssigkeit geeignet ist.

Das Hämodlalysegerät 10;10' weist eine Gerätesteuerung 40 auf, der eine Konservierungstaste 71 zugeordnet ist. Die Gerätesteuerung 40 steuert unter anderem einen Antriebsmotor 21 für die Flüssigkeitspumpe 22, der auch die Abfallpumpe 23 antreibt. Ferner steuert die Gerätesteuerung 40 insbesondere alle vorbeschriebenen Ventile 37,41,43,80 sowie die Flüssigkeitsheizung 30, und empfängt die von dem Konzentrations-Sensor 32 gelieferten Konzentrations-Werte.

Während der Dialysebehandlung eines Patienten ist an den geräteseitigen fluidischen Konnektoren 42,44 ein Wegwerf-Dialyseset 50 mit seinen korrespondierenden Konnektoren 43',45' angeschlossen, das den Dialysator 52 aufweist. Die Dialyseflüssigkeit wird von einer nicht dargestellten Dialysatquelle durch die Flüssigkeitspumpe 22 über die Dialysator-Zulaufleitung 17 zu dem Dialysator 52 und von dort über die Dialysator-Ablaufleitung 18 zu dem Abfallpfad 19 gepumpt, der in einen Abfalltank 62 für Flüssigabfälle mündet.

Nach der Dialysebehandlung wird das Wegwerf-Dialyseset 50 durch Trennen der betreffenden Konnektoren 42-45 entfernt und entsorgt. Anschließend erfolgt ein Desinfektions- und Konservierungszyklus zum Desinfizieren der Dialysatseite 12 des Hämodialysegeräts 10;10'.

Hierzu wird zunächst das Rückschlussset 14 gemäß dem ersten Ausführungsbeispiel bzw. das Rückschlussset 14' gemäß dem zweiten Ausführungsbeispiel durch verbinden der zugeordneten Konnektoren 42,43 bzw. 44,45 an die Dialysatseite 12 des Hämodialysegeräts 10;10' appliziert, Ferner wird der Desinfektionsflüssigkeits-Behälter 34;34' einschließlich des Desinfektionsventils 37 appliziert, wie in der Figur 1 bzw, in der Figur 2 dargestellt. Es wird ein Konservierungsflüssigkeits-Behälter 60 mit der Konservierungsflüssigkeit 61 an das Konservierungsventil 80 angeschlossen. Schließlich erhält die Gerätesteuerung 40 durch Betätigen der Konservierungstaste 71 ein Startsignal, wodurch der eigentliche Desinfektionsprozess gestartet wird.

Die Gerätesteuerung 40 öffnet das Desinfektionsventil 37 und das Rezirkulationsventil 43, und lässt das Konservierungsventil 80 geschlossen. Ferner aktiviert die Gerätesteuerung 40 die Flüssigkeitsheizung 30, startet den Antriebsmotor 21 der Flüssigkeitspumpe 22 und der Abfallpumpe 23 und startet auch die Ultrafiltrationspumpe 26. Die Gerätesteuerung 40 schaltet das Rezirkulationsventil 43 und das Abfallventil 41 während des Anlaufs der Desinfektion so, dass die zuvor in den Leitungen vorhandenen Dialyseflüssigkeit in den Abfallpfad 19 ausgeschoben und durch die Desinfektionsflüssigkeit, die aus dem Desinfektionsflüssigkeit-Behälter 34 einfließt, sukzessive und vollständig ersetzt wird. Sobald die Dialyseflüssigkeit vollständig ausgeschoben ist, wird das Desinfektionsventil 37 geschlossen, bleibt das Abfallventil 41 ständig geschlossen und bleibt das Rezirkulationsventil 43 ständig geöffnet. Die auf diese Weise in den Leitungen 15-18,27 durch die Flüssigkeitspumpe 22 zirkulierte Desinfektionsflüssigkeit wird hierbei durch die Flüssigkeitsheizung 30 auf eine Temperatur von 60° bis 80 °C aufgeheizt.

Nach Ablauf einer Desinfektionszeit von ca. 20 min wird die eigentliche Desinfektion beendet und in eine Konservierung überführt, Hierzu wird das Konservierungsventil 80 geöffnet und werden das Abfallventil 41 und das Rezirkulationsventil 43 gegensinnig zueinander geschaltet und regelmäßig umgeschaltet, so dass stets ein Ventil geöffnet und das andere geschlossen ist. Hierdurch wird jeweils ein Teil der systemischen Flüssigkeit in den Abfallpfad 19 und ein anderer Teil in die Rezirkulationsleitung 16 geleitet. Auf diese Weise wird die Desinfektionsflüssigkeit homogen verdünnt, bis die durch den Konzentrations-Sensor 32 gemessene Konzentration der Desinfektionsflüssigkeit in der Konservierungsflüssigkeit bei ca. 15 % liegt. Sobald die Konzentration der Desinfektionsflüssigkeit in der Konservierungsflüssigkeit einen Sollwert erreicht hat, werden alle Pumpen 42,23,26 gestoppt und alle Ventile 41,43,80,37; 37' geschlossen, womit das eigentliche Konservierungsintervall beginnt, das sich über mehrere Tage erstrecken kann.

Das Konservierungsintervall wird manuell durch erneutes Betätigen einer entsprechenden Taste, beispielsweise der Konservierungstaste 71, beendet. Hierdurch wird ein Reinigungsintervall gestartet, wozu die Pumpen 42,23,26 wieder gestartet, das Abfallventil 41 geöffnet und eine Spülflüssigkeit eingeleitet wird. Das Einleiten der Spülflüssigkeit kann dadurch erfolgen, dass die Konservierungsflüssigkeit 61, die aus Dialysewasser besteht und auch zum Spülen geeignet ist, über das geöffnete Konservierungsventil 80 in die Dialysator-Zulaufleitung 17 eingeleitet wird. Da auch die Rezirkulationsleitung 16 gespült werden muss, wird auch das Rezirkulationsventil 43 zeitweise geöffnet.

Durch den Konzentrations-Sensor 32 kann festgestellt werden, ob sich noch eine bedenkliche Konzentration an Desinfektionsflüssigkeit in dem Fluidkreis befindet. Sobald durch den Konzentrations-Sensor 32 eine unbedenkliche Desinfektionsflüssigkeits-Konzentration festgestellt wird oder aber nach einer definierten Spüldauer wird das Spülintervall dadurch beendet, dass alle Ventile 41,43,80,37; 37' geschlossen, die Flüssigkeitspumpen 23, 23,26 angehalten und schließlich das Rückschlussset 14; 14' entfernt wird.

Anschließend kann ein neues Wegwerf-Dialyseset 50 appliziert werden.

## Patentansprüche

1. Verfahren zum Desinfizieren einer Dialysatseite (12) eines Hämodialysegeräts (10;10'),
wobei das Hämodialysegerät (10;10') aufweist:
eine Dialysator-Zulaufleitung (17), die fluidisch in einen Zulaufkonnektor (42) mündet,
eine Dialysator-Ablaufleitung (18), die fluidisch an einem Ablaufkonnektor (44) beginnt,
eine Rezirkulations-Leitung (16) mit einem Rezirkulations-Ventil (43) zwischen der Dialysator-Ablaufleitung (18) und der Dialysator-Zulaufleitung (17),
eine temporäre Rückschlussleitung (15; 15') zwischen dem Zulauf-Konnektor (42) und dem Ablauf-Konnektor (44),
eine Desinfektionsflüssigkeit (36), die über ein Desinfektionsventil (37) in eine der Leitungen (15-18) eingeleitet werden kann,
eine Konservierungsflüssigkeit (61), die über ein Konservierungsventil (80) in eine der Leitungen (15-18) eingeleitet werden kann, und
eine Flüssigkeitspumpe (22) im Verlauf einer der Leitungen (15-18),
mit den Verfahrensschritten:
Einleiten der Desinfektionsflüssigkeit (36) in die Leitungen (15-18),
Öffnen des Rezirkulationsventils (43) und Aktivieren der Flüssigkeitspumpe (22) für eine vorgegebene Desinfektionszeit,
nach Ablauf der Desinfektionszeit: Öffnen des Konservierungsventils (80), wodurch die Konservierungsflüssigkeit (61) in alle Leitungen (15-18) eingeleitet wird, wobei die Konservierungsflüssigkeit (61) ein Verdünnungsmittel ist, mit dem die Desinfektionsflüssigkeit in den Leitungen (15-18) auf höchstens 50 % verdünnt wird, und
nach Ablauf einer Konservierungszeit: Öffnen eines Abfallventils (41), so dass die Konservierungsflüssigkeit aus den Leitungen (15-18) in eine Abfallleitung (19) gepumpt wird.

2. Verfahren zum Desinfizieren einer Dialysatseite (12) eines Hämodialysegeräts (10;10') nach einem der vorangegangenen Ansprüche, mit dem Verfahrensschritt:
nach dem Einleiten der Konservierungsflüssigkeit in die Leitungen (15-18): Anhalten der Flüssigkeitspumpe (22) für die Dauer der Konservierungszeit.

3. Verfahren zum Desinfizieren einer Dialysatseite (12) eines Hämodialysegeräts (10) nach einem der vorangegangenen Ansprüche, wobei ein Desinfektionsflüssigkeits-Behälter (34) und das Desinfektionsventil (37) der temporären Rückschlussleitung (15) fluidisch zugeordnet sind.

4. Verfahren zum Desinfizieren einer Dialysatseite (12) eines Hämodialysegeräts (10') nach einem der vorangegangenen Ansprüche 1 oder 2, wobei ein Desinfektionsflüssigkeits-Behälter (34') und das Desinfektionsventil (37') der Dialysator-Ablaufleitung (18) zugeordnet sind.

5. Verfahren zum Desinfizieren einer Dialysatseite (12) eines Hämodialysegeräts (10;10') nach einem der vorangegangenen Ansprüche, wobei die Konservierungsflüssigkeit (61) Dialysewasser ist.

6. Verfahren zum Desinfizieren einer Dialysatseite (12) eines Hämodialysegeräts (10) nach einem der vorangegangenen Ansprüche, wobei im Verlauf einer der geräteseitigen Leitungen (16-18), bevorzugt im Verlauf der der Dialysator-Zulaufleitung (17), ein Konzentrations-Sensor (32) zur Bestimmung der Konzentration der Desinfektionsflüssigkeit in der Konservierungsflüssigkeit angeordnet ist.

7. Verfahren zum Desinfizieren einer Dialysatseite (12) eines Hämodialysegeräts (10) nach einem der vorangegangenen Ansprüche, wobei das Hämodialysegerät (10; 10') eine Konservierungstaste (71) aufweist, durch deren Betätigung das Verfahren gemäß einer der vorangegangenen Ansprüche manuell ausgelöst wird.

8. Verfahren zum Desinfizieren einer Dialysatseite (12) eines Hämodialysegeräts (10) nach einem der vorangegangenen Ansprüche, mit dem Verfahrensschritt
nach dem Abpumpen der Konservierungsflüssigkeit in die Abfallleitung (19): Spülen der Leitungen (15-18) mit einer Spülflüssigkeit, wobei die Spülflüssigkeit bevorzugt Dialysewasser ist.

9. Verfahren zum Desinfizieren einer Dialysatseite (12) eines Hämodialysegeräts (10) nach einem der vorangegangenen Ansprüche, wobei im Verlauf der Dialysator-Zulaufleitung (17) eine Flüssigkeitsheizung (30) angeordnet ist, die während der Desinfektionszeit aktiviert ist.

## Claims

1. Method for disinfecting a dialysate side (12) of a hemodialysis unit (10; 10'), the hemodialysis unit (10; 10') comprising:
a dialyzer inflow line (17) fluidically opening into an inflow connector (42),
a dialyzer outflow line (18) fluidically starting at an outflow connector (44),
a recirculation line (16) with a recirculation valve (43) between the dialyzer outflow line (18) and the dialyzer inflow line (17),
a return line (15; 15') between the inflow connector (42) and the outflow connector (44),
a disinfection liquid (36) adapted to be introduced into one of the lines (15-18) via a disinfection valve (37),
a preservation liquid (61) adapted to be introduced into one of the lines (15-18) via a preservation valve (80), and
a liquid pump (22) arranged along one of the lines (15-18),
the method comprising the steps of:
introducing the disinfection liquid (36) into the lines (15-18),
opening the recirculation valve (43) and activating the liquid pump (22) for a predetermined disinfection period,
after the lapse of the disinfection period: opening the preservation valve (80), whereby the preservation liquid (61) is introduced into all lines (15-18), wherein the preservation liquid (61) is a diluent by which the disinfection liquid in the lines (15-18) is diluted to 50% at most, and after the lapse of a preservation period: opening a waste valve (41) so that the preservation liquid is pumped from the lines (15-18) into a waste line (19).

2. Method for disinfecting a dialysate side (12) of a hemodialysis unit (10; 10') of the preceding claim, the method comprising the step of:
after the introduction of the preservation liquid into the lines (15-18): stopping the liquid pump (22) for the duration of the preservation period.

3. Method for disinfecting a dialysate side (12) of a hemodialysis unit (10) of one of the preceding claims, wherein a disinfection liquid container (34) and the disinfection valve (7) are fluidically associated to the return line (15).

4. Method for disinfecting a dialysate side (12) of a hemodialysis unit (10') of one of the preceding claims 1 or 2, wherein a disinfection liquid container (34') and the disinfection liquid (37') are associated to the dialyzer outflow line (18).

5. Method for disinfecting a dialysate side (12) of a hemodialysis unit (10; 10') of one of the preceding claims, wherein the preservation liquid (61) is dialysis water.

6. Method for disinfecting a dialysate side (12) of a hemodialysis unit (10) of one of the preceding claims, wherein a concentration sensor (32) is arranged along one of the unit-side lines (16-18), preferably along the dialyzer inflow line (17), for determining the concentration of the disinfection liquid in the preservation liquid.

7. Method for disinfecting a dialysate side (12) of a hemodialysis unit (10) of one of the preceding claims, wherein the hemodialysis unit (10; 10') comprises a preservation key (71), the actuation of which manually initiates the method of one of the preceding claims.

8. Method for disinfecting a dialysate side (12) of a hemodialysis unit (10) of one of the preceding claims, the method comprising the step of:
after having pumped off the preservation liquid into the waste line (19): washing the lines (15-18) using a washing liquid, the washing liquid preferably being dialysis water.

9. Method for disinfecting a dialysate side (12) of a hemodialysis unit (10) of one of the preceding claims, wherein liquid heating (30) is arranged along the dialyzer inflow line (17), which is activated during the disinfection period.

## Revendications

1. Procédé de désinfection d'un côté dialysat (12) d'un appareil d'hémodialyse (10; 10'), ledit appareil d'hémodialyse (10; 10') comportant:
une conduite d'amenée (17) du dialyseur s'ouvrant fluidiquement dans un connecteur d'amenée (42),
une conduite de sortie (18) du dialyseur commençant fluidiquement à un connecteur de sortie (44),
une conduite de recirculation (16) avec une vanne de recirculation (43) entre la conduite de sortie (18) du dialyseur et la conduite d'amenée (17) du dialyseur,
une conduite de reflux (15; 15') temporaire entre le connecteur d'amenée (42) et le connecteur de sortie (44),
une liquide de désinfection (36) pouvant être introduite dans une des conduites (15-18) par une vanne de désinfection (37),
une liquide de conservation (61) pouvant être introduite dans une des conduites (15-18) par une vanne de conservation (80), et
une pompe à liquide (22) disposée sur le parcours d'une des conduites (15-18),
avec les étapes de procédé suivantes:
l'introduction du liquide de désinfection (36) dans les conduites (15-18),
l'ouverture de la vanne de recirculation (43) et l'activation de la pompe à liquide (22) pour un temps de désinfection prédéterminé,
à la fin du temps de désinfection: l'ouverture de la vanne de conservation (80), provoquant l'introduction du liquide de conservation (61) dans toutes les conduites (15 - 18), le liquide de conservation (61) étant un diluant par lequel le liquide de désinfection est dilué dans les conduites (15-18) à 50% au maximum, et
à la fin d'un temps de conservation: l'ouverture d'une vanne de déchets (41), de sorte que le liquide de conservation soit pompé des conduites (15-18) dans une conduite de déchets (19(.

2. Procédé de désinfection d'un côté dialysat (12) d'un appareil d'hémodialyse (10; 10') selon la revendication précédente, le procédé comportant l'étape suivante:
après l'introduction du liquide de conservation dans les conduites (15-18): arrêter la pompe á liquide (22) pour la durée du temps de conservation.

3. Procédé de désinfection d'un côté dialysat (12) d'un appareil d'hémodialyse (10), selon l'une quelconque des revendications précédentes, dans lequel un réceptacle de liquide de désinfection (34) et la vanne de désinfection (37) sont associés fluidiquement à la conduite de reflux (15) temporaire.

4. Procédé de désinfection d'un côté dialysat (12) d'un appareil d'hémodialyse (10'), selon l'une quelconque des revendications précédentes 1 ou 2, dans lequel un réceptacle de liquide de désinfection (34') et a vanne de désinfection (37') sont associés à la conduite de sortie (18) du dialyseur.

5. Procédé de désinfection d'un côté dialysat (12) d'un appareil d'hémodialyse (10; 10') selon la revendication précédente, dans lequel le liquide de conservation (61) est de l'eau de dialyse.

6. Procédé de désinfection d'un côté dialysat (12) d'un appareil d'hémodialyse (10), selon l'une quelconque des revendications précédentes, dans lequel sur le parcours d'une des conduite (16-18), côté appareil, de préférence sur le parcours de la conduite d'amenée (17) du dialyseur, un capteur de concentration (32) est disposé pour déterminer la concentration en liquide de désinfection dans le liquide de conservation.

7. Procédé de désinfection d'un côté dialysat (12) d'un appareil d'hémodialyse (10), selon l'une quelconque des revendications précédentes, dans lequel l'appareil d'hémodialyse (10; 10') comprend un bouton de conservation (71) dont l'actionnement manuellement provoque le procédé selon l'une des revendications précédentes.

8. Procédé de désinfection d'un côté dialysat (12) d'un appareil d'hémodialyse (10), selon l'une quelconque des revendications précédentes, avec l'étape de procédé suivante:
après le pompage du liquide de conservation dans la conduite de déchets (19): rinçage des conduite (15-18) avec un liquide de rinçage, le liquide de rinçage étant de préférence de l'eau de dialyse.

9. Procédé de désinfection d'un côté dialysat (12) d'un appareil d'hémodialyse (10), selon l'une quelconque des revendications précédentes, dans lequel un chauffage de liquide (30) est disposée sur le parcours de la conduite d'amenée (17) du dialyseur, le chauffage étant activé pendant le temps de désinfection.
